(19) Europäisches Patentamt
European Patent Office
Office européen des brevets

(11) Veröffentlichungsnummer: **0 565 986 A2**

(12) # EUROPÄISCHE PATENTANMELDUNG

(21) Anmeldenummer: **93105613.9**

(22) Anmeldetag: **05.04.93**

(51) Int. Cl.[5]: **C07D 233/68**, A61K 31/415, //C07D233/66,C07D233/54

(30) Priorität: **16.04.92 DE 4212796**

(43) Veröffentlichungstag der Anmeldung: **20.10.93 Patentblatt 93/42**

(84) Benannte Vertragsstaaten: **AT BE CH DE DK ES FR GB GR IE IT LI LU MC NL PT SE**

(71) Anmelder: **BAYER AG**

**D-51368 Leverkusen(DE)**

(72) Erfinder: **Müller-Gliemann, Matthias, Dr.**
**Laibacher Strasse 10**
**W-5650 Solingen(DE)**
Erfinder: **Dressel, Jürgen**
**Claudiusweg 9**
**W-5600 Wuppertal 1(DE)**
Erfinder: **Fey, Peter, Dr.**
**Am Eickhof 23**
**W-5600 Wuppertal 1(DE)**
Erfinder: **Hanko, Rudolf, Dr.**
**Schillerstrasse 23**
**W-4000 Düsseldorf 1(DE)**
Erfinder: **Hübsch, Walter, Dr.**
**Wildsteig 22**
**W-5600 Wuppertal 1(DE)**
Erfinder: **Krämer, Thomas, Dr.**
**In den Birken 92a**
**W-5600 Wuppertal 1(DE)**
Erfinder: **Müller, Ulrich, Dr.**
**Claudiusweg 5**
**W-5600 Wuppertal 1(DE)**
Erfinder: **Beuck, Martin, Dr.**
**Trills 7**
**W-4006 Erkrath 2(DE)**
Erfinder: **Kazda, Stanislav, Prof. Dr.**
**Gellertweg 18**
**W-5600 Wuppertal 1(DE)**
Erfinder: **Knorr, Andreas, Dr.**
**Trillser Graben 10**
**W-4006 Erkrath 2(DE)**
Erfinder: **Stasch, Johannes-Peter, Dr.**
**Alfred-Nobel Strasse 109**
**D-42651 Solingen(DE)**
Erfinder: **Wohlfeil, Stefan, Dr.**
**Tucherweg 25**
**W-4010 Hilden(DE)**

(54) **Propenoyl-imidazolderivate, ihre Herstellung und ihre Verwendung als Medikamente mit Angiotensin II-antagonistischer Wirkung.**

(57) Propenoyl-imidazolderivate werden durch Umsetzung von entsprechenden Imidazoylaldehyden mit CH-aciden Verbindungen und anschließende Dehydratisierung der Zwischenprodukte hergestellt. Die Propenoyl-imidazolderivate können als Wirkstoffe in Arzneimitteln zur Behandlung der arteriellen Hypertonie und Atherosklerose eingesetzt werden.

EP 0 565 986 A2

Die Erfindung betrifft Propenoyl-imidazolderivate, Verfahren zu ihrer Herstellung, sowie ihre Verwendung in Arzneimitteln, insbesondere als blutdrucksenkende und antiatherosklerotische Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksteigernden Oktapeptid Angiotensin II abgebaut wird. Die verschiedenen Effekte des Angiotensin II, wie beispielsweise Vasokonstriktion, $Na^+$-Retention in der Niere, Aldosteronfreisetzung in der Nebenniere und Tonuserhöhung des sympathischen Nervensystems wirken synergistisch im Sinne einer Blutdruckerhöhung.

Darüber hinaus besitzt Angiotensin II die Eigenschaft, das Wachstum und die Vermehrung von allen wie beispielsweise von Herzmuskelzellen und glatten Muskelzellen zu fördern, wobei diese bei verschiedenen Krankheitszustanden (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Die Erfindung betrifft Propenoyl-imidazolderivate der allgemeinen Formel (I)

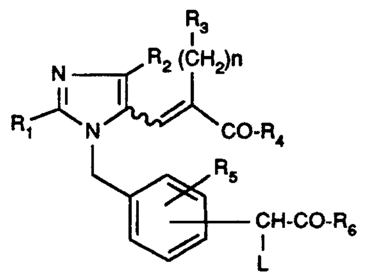

(I)

in welcher

| | |
|---|---|
| $R^1$ | für geradkettiges oder verzweigtes Alkyl oder Alkenyl mit jeweils bis zu 8 Kohlenstoffatomen steht, oder<br>für Cycloalkyl mit 3 bis 8 Kohlenstoffatomen steht, |
| $R^2$ | für Wasserstoff, Halogen oder für Perfluoralkyl mit bis zu 5 Kohlenstoffatomen steht, |
| n | für eine Zahl 0, 1, 2 oder 3 steht, |
| $R^3$ | für Cycloalkyl mit 3 bis 7 Kohlenstoffatomen steht, |
| $R^4$ | für Hydroxy, für geradkettiges oder verzweigtes Alkoxy mit bis zu 6 Kohlenstoffatomen steht, oder<br>für einen Rest der Formel $-CO-NR^7R^8$ steht,<br>worin |
| $R^7$ und $R^8$ | gleich oder verschieden sind und Wasserstoff, geradkettiges oder verzweigtes Alkyl mit bis zu 6 Kohlenstoffatomen oder Phenyl bedeuten, |
| $R^5$ | für Wasserstoff, Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano oder Carboxy steht, |
| L | für Cycloalkyl mit 3 bis 7 Kohlenstoffatomen steht, oder für geradkettiges oder verzweigtes Alkyl mit bis zu 8 Kohlenstoffatomen steht, das gegebenenfalls durch Phenyl substituiert ist, |
| $R^6$ | für Hydroxy oder geradkettiges oder verzweigtes Alkoxy mit bis zu 6 Kohlenstoffatomen steht, oder<br>für einen Rest der Formel $-NR^9SO_2R^{10}$ oder |

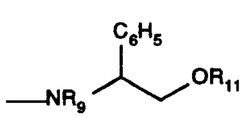

steht,

worin

$R^9$ Wasserstoff oder geradkettiges oder verzweigtes Alkyl mit bis zu 4 Kohlenstoffatomen bedeutet,

$R^{10}$ geradkettiges oder verzweigtes Alkyl mit bis zu 6 Kohlenstoffatomen oder Phenyl bedeutet, das gegebenenfalls durch geradkettiges oder verzweigtes Alkyl mit bis zu 6 Kohlenstoffatomen substituiert ist,

$R^{11}$ Wasserstoff, geradkettiges oder verzweigtes Alkyl mit bis zu 6 Kohlenstoffatomen oder eine Hydroxyschutzgruppe bedeutet

und deren Salze.

Die erfindungsgemäßen Verbindungen der allgemeinen Formel (I) können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der Propenoyl-imidazolderivate können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweilige Mischungen. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bevorzugt sind Verbindungen der allgemeinen Formel (I),

in welcher

R $^1$ für geradkettiges oder verzweigtes Alkyl oder Alkenyl mit jeweils bis zu 6 Kohlenstoffatomen steht, oder
für Cyclopropyl, Cyclobutyl, Cyclopentyl, Cyclohexyl oder Cycloheptyl steht,

$R^2$ für Wasserstoff, Fluor, Chlor, Brom oder für Perfluoralkyl mit bis zu 4 Kohlenstoffatomen steht,

n für eine Zahl 0, 1 oder 2 steht,

$R^3$ für Cyclopropyl, Cyclobutyl, Cyclopentyl, Cyclohexyl oder Cycloheptyl steht,

$R^4$ für Hydroxy, für geradkettiges oder verzweigtes Alkoxy mit bis zu 4 Kohlenstoffatomen, oder
für die Gruppe der Formel $-CO-NR^7R^8$ steht,
worin

$R^7$ und $R^8$ gleich oder verschieden sind und Wasserstoff, geradkettiges oder verzweigtes Alkyl mit bis zu 4 Kohlenstoffatomen oder Phenyl bedeuten,

$R^5$ für Wasserstoff, Fluor, Chlor, Brom, Trifluormethyl, Carboxy oder geradkettiges oder verzweigtes Alkyl, Alkoxy oder Alkoxycarbonyl mit jeweils bis zu 4 Kohlenstoffatomen steht,

L für Cyclopropyl, Cyclobutyl, Cyclopentyl, Cyclohexyl oder Cycloheptyl steht, oder
für geradkettiges oder verzweigtes Alkyl mit bis zu 6 Kohlenstoffatomen oder für Benzyl steht,

$R^6$ für Hydroxy oder für geradkettiges oder verzweigtes Alkoxy mit bis zu 4 Kohlenstoffatomen steht, oder
für einen Rest der Formel $-NR^9SO_2R^{10}$ oder

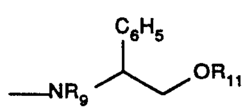

steht,
worin

$R^9$ Wasserstoff, Methyl oder Ethyl bedeutet,

$R^{10}$ geradkettiges oder verzweigtes Alkyl mit bis zu 4 Kohlenstoffatomen oder Phenyl bedeutet, das gegebenenfalls durch geradkettiges oder verzweigtes Alkyl mit bis zu 4 Kohlenstoffatomen substituiert ist,

$R^{11}$ Wasserstoff, geradkettiges oder verzweigtes Alkyl mit bis zu 4 Kohlenstoffatomen, Acetyl oder Benzyl bedeutet,

und deren Salze.

Besonders bevorzugt sind Verbindungen der allgemeinen Formel (I),
in welcher

$R^1$ für geradkettiges oder verzweigtes Alkyl oder Alkenyl mit jeweils bis zu 4 Kohlenstoffatomen steht, oder für Cyclopropyl, Cyclopentyl oder Cyclohexyl steht,

$R^2$ für Wasserstoff, Fluor, Chlor oder für Perfluoralkyl mit bis zu 2 Kohlenstoffatomen steht,

n für die Zahl 0 oder 1 steht,

$R^3$ für Cyclopentyl oder Cyclohexyl steht,

$R^4$ für Hydroxy, Methoxy, Ethoxy oder tert.Butoxy steht,

$R^5$ für Wasserstoff, Fluor, Chlor, Brom, Trifluormethyl, Trifluormethoxy oder Methyl steht,

L für Cyclopentyl, Cyclohexyl oder Cycloheptyl steht, oder
für geradkettiges oder verzweigtes Alkyl mit bis zu 4 Kohlenstoffatomen oder für Benzyl steht,

$R^6$ für Hydroxy oder für geradkettiges oder verzweigtes Alkoxy mit bis zu 4 Kohlenstoffatomen steht, oder
für einen Rest der Formel $-NR^9SO_2R^{10}$ oder

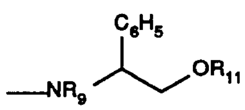

steht,
worin

$R^9$ Wasserstoff oder Methyl bedeutet,

$R^{10}$ Methyl oder p-Tolyl bedeutet,

$R^{11}$ Wasserstoff oder geradkettiges oder verzweigtes Alkyl mit bis zu 3 Kohlenstoffatomen oder Benzyl bedeutet,

und deren Salze.

Ganz besonders bevorzugt sind die erfindungsgemäßen Verbindungen der allgemeinen Formel (I), in welcher der Rest $-CH(L)-CO-R^6$ in para-Position zur Imidazolylmethylgruppe steht.

Außerdem wurde ein Verfahren zur Herstellung der erfindungsgemäßen Verbindungen der allgemeinen Formel (I) gefunden, dadurch gekennzeichnet, daß man
Aldehyde der allgemeinen Formel (II)

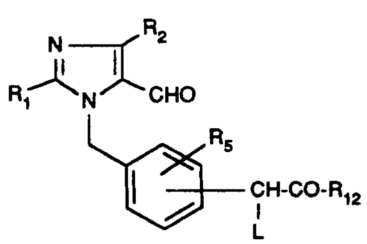

(II)

in welcher

$R^1$, $R^2$, $R^5$ und L die oben angegebene Bedeutung haben

und

$R^{12}$ für $C_1$-$C_4$-Alkoxy steht,

durch Umsetzung mit Verbindungen der allgemeinen Formel (III)

$R^3$-$(CH_2)_n$-$CH_2$-CO-$R^{13}$ (III)

in welcher

$R^3$ und n die oben angegebene Bedeutung haben

und

$R^{13}$ die oben angegebene Bedeutung von $R^4$ hat aber nicht für Hydroxy steht,

in inerten Lösemitteln, in Anwesenheit einer Base in die Hydroxy-Verbindungen der allgemeinen Formel (IV)

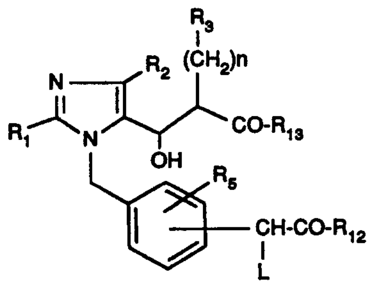

(IV)

in welcher

$R^1$, $R^2$, $R^3$, $R^5$, $R^{12}$, $R^{13}$, n und L die oben angegebene Bedeutung haben,

überführt,

anschließend die freie Hydroxyfunktion durch Einführung einer Schutzgruppe blockiert, in einem letzten Schritt eine Eliminierung in inerten Lösemitteln, in Anwesenheit einer Base durchführt, gegebenenfalls die E/Z-Isomeren trennt,

und im Fall der Säuren ($R^4$ = OH) die Ester verseift,

und im Fall der Amide oder Sulfonamide, gegebenenfalls nach Aktivierung der Carbonsäure ($R^{12}$ = OH) eine Amidierung oder Sulfoamidierung nach üblichen Methoden anschließt,

im Fall, daß $R^9$ nicht für Wasserstoff steht, die NH-Funktion alkyliert,

und gegebenenfalls die Substituenten $R^1$ und $R^2$ nach üblichen Methoden, beispielsweise durch Reduktion, Oxidation, Alkylierung oder Hydrolyse einführt oder in andere Gruppen überführt

und gegebenenfalls die Stereoisomeren trennt, und im Fall der Herstellung der Salze mit einer entsprechenden Base oder Säure umsetzt.

Das erfindungsgemäße Verfahren kann durch folgendes Formelschema beispielhaft erläutert werden:

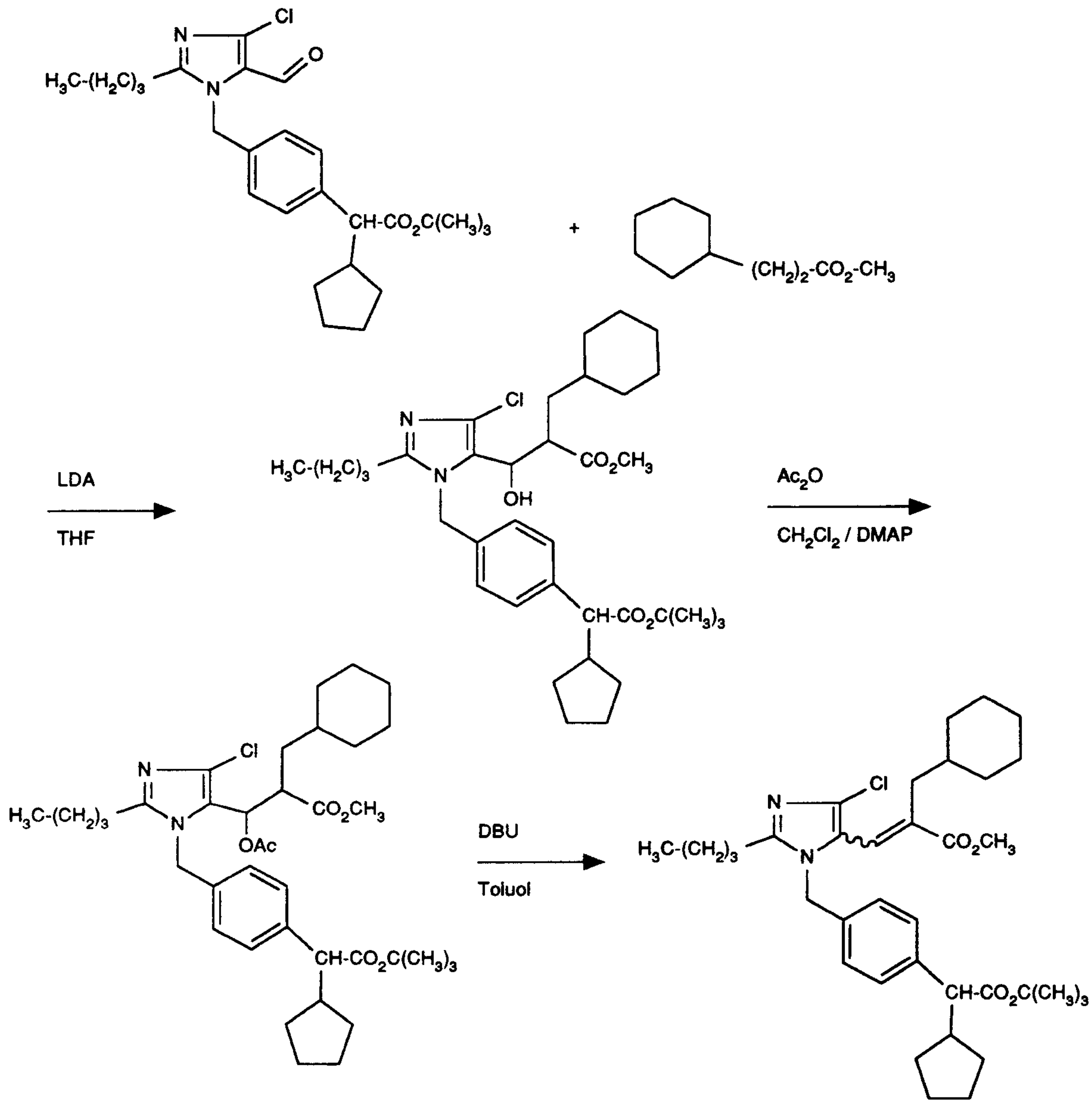
+
LDA
THF
Ac2O
CH2Cl2 / DMAP
DBU
Toluol

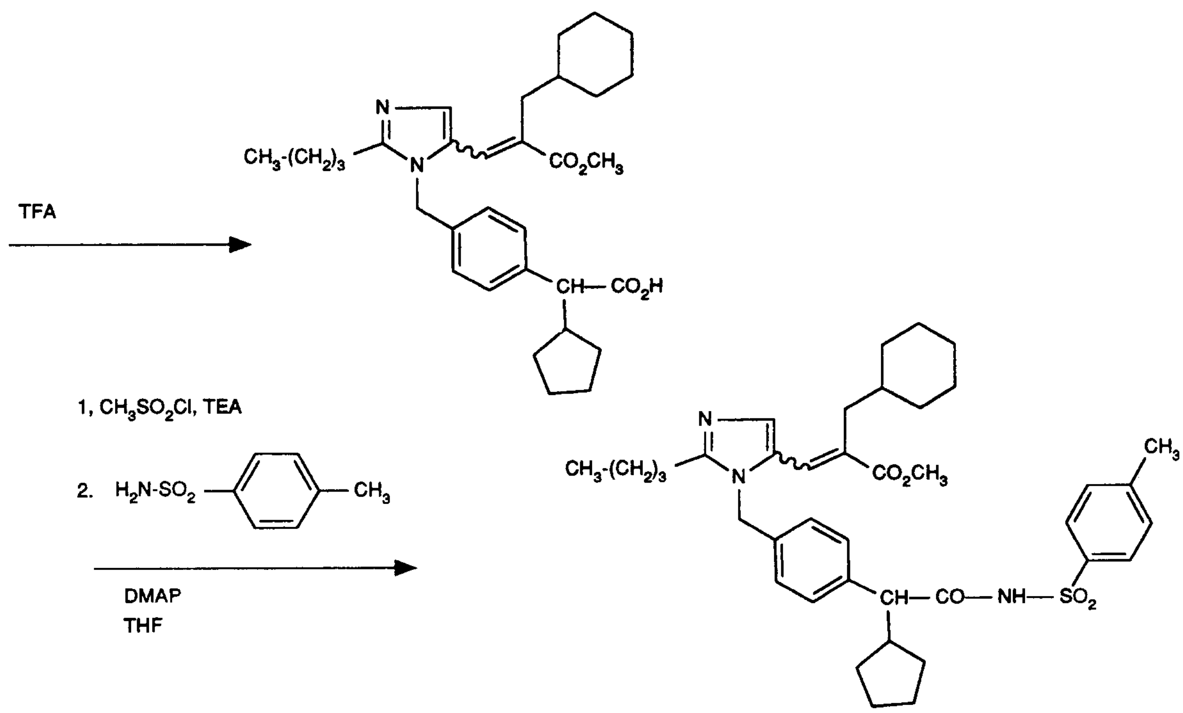


Hydroxyschutzgruppe im Rahmen der oben angegebenen Definition steht im allgemeinen für eine Schutzgruppe aus der Reihe: Benzyloxycarbonyl, Methansulfonyl, Toluolsulfonyl, 2-Nitrobenzyl, 4-Nitrobenzyl, 2-Nitrobenzyloxycarbonyl, 4-Nitrobenzyloxycarbonyl, tert.Butoxycarbonyl, Allyloxycarbonyl, 4-Methoxycarbonyl, Acetyl, Trichloracetyl, 2,2,2-Trichlorethoxycarbonyl, 2,4-Dimethoxybenzyloxycarbonyl, 2-(Methylthiomethoxy)ethoxycarbonyl, Benzoyl, 4-Methylbenzoyl, 4-Nitrobenzoyl, 4-Fluorbenzoyl, 4-Chlorbenzoyl oder 4-Methoxybenzoyl. Bevorzugt sind Acetyl, Methansulfonyl und Toluolsulfonyl.

Als Lösemittel für das Verfahren eignen sich übliche organische Losemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind für die verschiedenen Schritte Tetrahydrofuran, Methylenchlorid, Toluol und Dioxan.

Als Basen für das erfindungsgemäße Verfahren können im allgemeinen anorganische oder organische Basen eingesetzt werden. Hierzu gehören vorzugsweise Alkali- oder Erdalkalihydroxide wie zum Beispiel Natriumhydroxid, Kaliumhydroxid, Lithiumhydroxid oder Bariumhydroxid, Alkalicarbonate wie Natriumcarbonat oder Kaliumcarbonat, Erdalkalicarbonate wie Calciumcarbonat, oder Alkali- oder Erdalkalialkoholate oder -amide wie Natrium- oder Kaliummethanolat, Natrium- oder Kaliumethanolat oder Kalium-tert.butylat oder Lithiumdiisopropylamid (LDA), oder n-Butyllithium, oder organische Amine (Trialkyl($C_1$-$C_6$)amine) wie Triethylamin oder N,N-Diisopropylamin, oder Heterocyclen wie 1,4-Diazabicyclo[2.2.2]octan (DABCO), 1,8-Diazabicyclo[5.4.0]undec-7-en (DBU), Pyridin, N,N-Dimethylaminopyridin (DMAP), Diaminopyridin, Methylpiperidin oder Morpholin. Es ist auch möglich, als Basen Alkalimetalle, wie Natrium oder deren Hydride wie Natriumhydrid, einzusetzen. Bevorzugt sind Triethylamin, Lithiumhydroxid, DBU, N,N-Dimethylaminopyridin und n-Butyllithium.

Im allgemeinen setzt man die Base in einer Menge von 0,05 mol bis 10 mol, bevorzugt von 1 mol bis 2 mol bezogen auf 1 mol der Verbindung der Formel (III) ein.

Die einzelnen Schritte des erfindungsgemäßen Verfahrens werden im allgemeinen in einem Temperaturbereich von -78 °C bis +80 °C, bevorzugt von -40 °C bis Raumtemperatur durchgeführt. Gegebenenfalls ist es erforderlich, unter Schutzgasatmosphäre zu arbeiten.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Einführung der Schutzgruppe erfolgt im allgemeinen in einem der oben aufgeführten Lösemitteln und einer Base,vorzugsweise in Methylenchlorid mit Dimethylaminopyridin.

Die Blockierung erfolgt im allgemeinen in einem Temperturbereich von 0°C bis +60°C, vorzugsweise bei Raumtemperatur und bei Normaldruck.

Die Eliminierung wird im allgemeinen in einem der oben aufgeführten Lösemittel, vorzugsweise in Toluol und in Anwesenheit einer der aufgeführten Basen, vorzugsweise DBU durchgeführt.

Die Eliminierung erfolgt im allgemeinen in einem Temperaturbereich von +30°C bis +130°C, vorzugsweise bei +50°C bis +100°C und Normaldruck.

Als Basen eignen sich für die Verseifung die üblichen anorganischen Basen. Hierzu gehören bevorzugt Alkalihydroxide oder Erdalkalihydroxide wie beispielsweise Lithiumhydroxid, Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Lithiumhydroxid, Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Dioxan-Wasser-Gemische eingesetzt.

Die Verseifung kann auch mit Säuren wie beispielsweise Trifluoressigsäure (TFA), Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Bei der Durchführung der Reaktion entstehen im ersten Schritt die Carboxylate der erfindungsgemäßen Verbindungen als Zwischenprodukte, die isoliert werden können. Die erfindungsgemäßen Säuren erhält man durch Behandeln der Carboxylate mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren hierbei als vorteilhaft erwiesen, die basische Reaktionsmischung der Verseifung in einem zweiten Schritt ohne Isolierung der Carboxylate anzusäuern. Die Säuren können dann in üblicher Weise isoliert werden.

Die Amidierung und die Sulfoamidierung erfolgen im allgemeinen in einem der oben aufgeführten Lösemittel, vorzugsweise in Tetrahydrofuran oder Dichlormethan.

Die Amidierung und die Sulfoamidierung können gegebenenfalls über die aktivierte Stufe der Säurehalogenide, die aus den entsprechenden Säuren durch Umsetzung mit Thionylchlorid, Phosphortrichlorid, Phosphorpentachlorid, Phosphortribromid oder Oxalylchlorid hergestellt werden können, verlaufen.

Die Amidierung und die Sulfoamidierung erfolgen im allgemeinen in einem Temperaturbereich von -20°C bis +80°C, vorzugsweise von - 10°C bis +30°C und Normaldruck.

Als Basen eignen sich dafür neben den oben aufgeführten Basen vorzugsweise Triethylamin und/oder Dimethylaminopyridin, DBU, DABCO oder N,N-Dimethylaminopyridin.

Die Base wird in einer Menge von 0,5 mol bis 10 mol, bevorzugt von 1 mol bis 5 mol, bezogen auf 1 mol der Verbindungen der allgemeinen Formeln (IV) und (V) eingesetzt.

Als säurebindende Mittel für die Amidierung können Alkali- oder Erdalkalicarbonate wie Natriumcarbonat, Kaliumcarbonat, Alkali- oder Erdalkalihydroxide wie beispielsweise Natrium- oder Kaliumhydroxid, oder organische Basen wie Pyridin, Triethylamin, N-Methylpiperidin, oder bicyclische Amidine wie 1,5-Diazabicyclo[3.4.0]-nonene-5 (DBN) oder 1,5-Diazabicyclo[3.4.0]undecene-5 (DBU) eingesetzt werden. Bevorzugt ist Kaliumcarbonat.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium-hexafluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid, N-Hydroxybenzotriazol oder N-Hydroxysuccinimid [vgl. J.C.

Sheehan, S.L. LEdis, J. Am. Chem. Soc. 95, 875 (1973); F.E. Frerman et al., J. Biol. Chem. 225, 507 (1982) und N.B. Benoton, K. Kluroda, Int. Pept. Prot. Res. 13, 403 (1979), 17, 187 (1981)]. Bevorzugt ist N,N-Dicyclohexylcarbodiimid, gegebenenfalls in Anwesenheit von Triethylamin und N-Hydroxybenzotriazol.

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren eingesetzt.

Die Verbindungen der allgemeinen Formel (II) sind neu und können hergestellt werden, indem man Verbindungen der allgemeinen Formel (V)

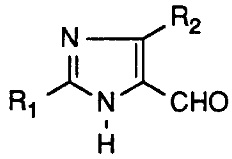

(V)

in welcher

$R^1$ und $R^2$ die oben angegebene Bedeutung haben, mit Verbindungen der allgemeinen Formel (VI)

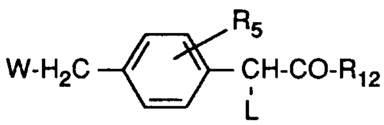

(VI)

in welcher

$R^5$, L und $R^{12}$ die oben angegebene Bedeutung haben

und

W für Halogen, vorzugsweise für Brom steht,

in einem der oben aufgeführten Lösemittel und in Anwesenheit einer der dort aufgeführten Basen, vorzugsweise in Dimethylformamid mit Natriumhydrid oder Kaliumcarbonat umsetzt.

Die Verbindungen der allgemeinen Formel (III) sind an sich bekannt oder können nach üblichen Methoden hergestellt werden.

Die Verbindungen der allgemeinen Formel (IV) sind neu und können nach dem oben aufgeführten Verfahren beispielsweise hergestellt werden.

Die Imidazole der allgemeinen Formel (V) sind bekannt oder in Analogie zu literaturbekannten Verfahren herstellbar (vgl. z.B. Beilstein 25, 163; 23 45, US 43 550 40].

Die Verbindungen der allgemeinen Formel (VI) sind teilweise bekannt und können beispielsweise hergestellt werden, indem man Verbindungen der allgemeinen Formel (VII)

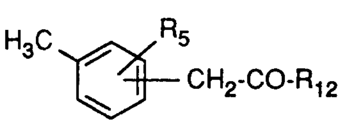

(VII)

in welcher

$R^5$ und $R^{12}$ die oben angegebene Bedeutung haben,

zunächst mit Verbindungen der allgemeinen Formel (VIII)

L-Z (VIII)

in welcher

L die oben angegebene Bedeutung hat,

und

Z für Halogen, vorzugsweise für Brom steht,

in inerten Lösemitteln, gegebenenfalls in Anwesenheit einer Base alkyliert,

und in einem zweiten Schritt an der Methylgruppe eine Bromierung, gegebenenfalls in Anwesenheit eines Katalysators, nach üblicher Methode durchführt.

Die Alkylierung erfolgt im allgemeinen in einem der oben aufgeführten Lösemitteln, vorzugsweise in Dimethylformamid in einem Temperaturbereich von 0°C bis +70°C, vorzugsweise von 0°C bis +30°C und Normaldruck.

Als Starter (Katalysator) für die Bromierung eignen sich beispielsweise Azobisisobutyronitril, Dibenzoylperoxid, vorzugsweise Azobisisobutyronitril, wobei der Starter in einer Menge von 0,01 mol bis 0,1 mol, bevorzugt von 0,01 mol bis 0,05 mol, bezogen auf 1 mol der Verbindung der allgemeinen Formel (VI) eingesetzt wird.

Die Verbindungen der allgemeinen Formel (VII) sind an sich bekannt oder können nach bekannten Methoden hergestellt werden [vgl. J. Chem. Soc., Perkin Trans. 1, (9), 1706 - 1707; J. Chem. Soc., Chem. Commun., (2), 167 - 168].

Die Verbindungen der allgemeinen Formel (VIII) sind an sich bekannt [vgl. Beilstein 5, 19/5, 24/5, 29] oder können nach üblicher Methode aus den entsprechenden Alkoholen oder Cycloalkenen hergestellt werden.

Die erfindungsgemäßen Verbindungen der allgemeinen Formel (I) zeigen ein nicht vorhersehbares, wertvolles pharmakologisches Wirkspektrum.

Die erfindungsgemäßen Verbindungen besitzen eine spezifische A II-antagonistische Wirkung, da sie die Bindung von Angiotensin II an A II-$AT_1$-Rezeptoren, an A II-$AT_2$-Rezeptoren oder an A II-$AT_1$ und gleichzeitig $AT_2$-Rezeptoren hemmen. Sie unterdrücken die vasokonstriktorischen und Aldosteronsekretionsstimulierenden Effekte des Angiotensin II. Darüberhinaus inhibieren sie die Proliferation von glatten Muskelzellen.

Sie können deshalb in Arzneimitteln zur Behandlung der arteriellen Hypertonie und Atherosklerose eingesetzt werden. Darüberhinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogenbegaster Krebs-Henseleit-Nährlösung folgender Zusammensetzung: 119 mmol/l NaCl; 2,5 mmol/l $CaCl_2$ x 2 $H_2O$; 1,2 mmol/l $KH_2PO_4$; 10 mmol/l Glucose; 4,8 mmol/l KCl; 1,4 mmol/l $MgSO_4$ x 7 $H_2O$ und 25 mmol/l $NaHCO_3$ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

**Agonisten und ihre Standardkonzentrationen (Applikationsvolumen pro Einzelgabe = 100 µl):**

| | | |
|---|---|---|
| KCl | 22,7;32,7;42,7;52,7 | mmol/l |
| l-Noradrenalin | $3x10^{-9}$;$3x10^{-8}$;$3x10^{-7}$;$3x10^{-6}$ | g/ml |
| Serotonin | $10^{-8}$;$10^{-7}$;$10^{-6}$;$10^{-5}$ | g/ml |
| B-HT 920 | $10^{-7}$;$10^{-6}$;$10^{-5}$ | g/ml |
| Methoxamin | $10^{-7}$;$10^{-6}$;$10^{-5}$ | g/ml |
| Angiotensin II | $3x10^{-9}$;$10^{-8}$;$3x10^{-8}$;$10^{-7}$ | g/ml |

Für die Berechnung der $IC_{50}$ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

**Tabelle A:**

**Hemmung der Gefäßkontraktion an isolierten Aortenringen von Kaninchen in vitro**

**$IC_{50}$ (g/ml) gegen Kontraktionen, induziert durch Angiotensin II:**

| Bsp.Nr.: | $IC_{50}$ [g/ml] |
|---|---|
| 4 | $6,3 \times 10^{-8}$ |
| 7 | $5,1 \times 10^{-8}$ |
| 12 | $4,6 \times 10^{-8}$ |

Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht. Die Blutdruckveränderungen unter Substanzeinfluß sind als Mittelwerte ± SEM in der Tabelle angegeben.

Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht.

Der arterielle Blutdruck diese Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

Interaktion der erfindungsgemäßen Verbindungen mit dem Angiotensin I-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.

Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 $\mu$g), $^3$H-Angiotensin II (3-5 nM), Test-Pufferlösung (50 mM Tris, pH 7,2), 5 mM $MgCl_2$ sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu $K_i$- bzw. $IC_{50}$-Werten ($K_i$: für die verwendete Radioaktivität korrigierte $IC_{50}$-Werte; $IC_{50}$-Werte: Konzentration bei der die zu untersuchende Substanz eine 50%ige Hemmung der spezifischen Bindung des Radioliganden bewirkt).

Bsp. 6 $K_i$ = 750 nM
Bsp. 8 $K_i$ = 80 nM

**Interaktion der erfindungsgemäßen Verbindungen mit dem Typ 2 Angiotensin II-Rezeptor ($AT_2$) an Membranfraktionen des Rinder-Kleinhirns**

Für den Test wird das $AT_2$-Rezeptor "Drug Discovery System" der Firma NEN-DuPont (Katalog Nr. NED-001) verwendet. Die Testdurchführung erfolgt entsprechend mit dem mitgelieferten Versuchsprotokoll. Die Rezeptor-Bindung wird in einem Testvolumen von 235 $\mu$l durchgeführt, das im einzelnen $^{125}$J-Angiotensin II (ca. 0,1 nM), Testpuffer (PBS, NaCl, EDTA, PMSF, DTT, DMSO) sowie die zu untersuchenden Substanzen enthält. Nach einer Inkubation von 60 min bei 37°C wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (0,9%ige NaCl) in einem Szintillationscocktail spektrophotometrisch gemessen. Die Analyse der Rohdaten erfolgte mit Computerprogrammen zur $IC_{50}$- bzw. $K_i$-Wert Bestimmung ($IC_{50}$-Wert: Konzentration bei der die zu untersuchende Substanz eine 50%ige Hemmung der spezifischen Bindung des Radioliganden bewirkt: $K_i$-Wert: für die verwendete Radioaktivität korrigierter $IC_{50}$-Wert).

Bsp.-Nr. 4    $K_i$(nM) = 827

Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium mit Serumzuwachs, 2 mMol L-Glutamin und 15 mMol HEPES, pH 7,4 in 5% $CO_2$ bei 37°C kultiviert. Danach werden die Zellen durch Serumentzug für 2 - 3 Tage synchronisiert und sodann mit Serum oder anderen Faktoren zum Wachstum angeregt. Gleichzeitig werden Testverbindungen zugesetzt. Nach 16 - 20 Stunden wird 1 $\mu$Ci $^3$H-Thymidin zugefügt und nach weiteren 4 Stunden der Einbau dieser Substanz in die TCA-präzipitierbare DNA der Zellen bestimmt.

Zur Bestimmung der $IC_{50}$-Werte wird die Wirkstoffkonzentration errechnet, die bei sequentieller Verdünnung des Wirkstoffes halbmaximale Hemmung der durch 10 % FCS hervorgerufene Thymidininkorporation bewirkt.

| Bsp.-Nr. | % Hemmung bei $10^{-6}$ M |
|---|---|
| 5 | 90 |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Lösemittelgemische

A: Essigsäureethylester / Petrolether 40-60 = 7:3
B: Dichlormethan / Methanol = 9:1
C: Dichlormethan / Methanol / Eisessig = 9:1:0,1
D: Essigsäureethylester/Petrolether 40-60 = 1:1
E: Toluol/Essigsäureethylester = 1:3

Ausgangsverbindungen

Beispiel I

2-[4-{2-n-Butyl-4-chlor-5-(1-(3-cyclohexyl-1-hydroxy-2-methoxycarbonyl)propyl)-1H-imidazol-1-yl-methyl}phenyl]-2-cyclopentyl-essigsäuretert.butylester

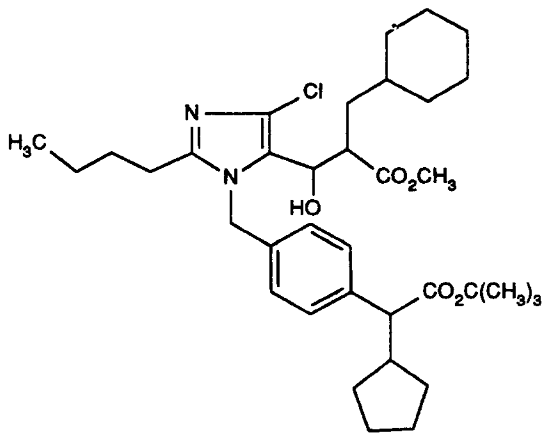

Zu einer Lösung von 4,4 g (44 mmol) Diisopropylamin in 50 ml Tetrahydrofuran werden unter Argon bei -78°C 25,9 ml einer 1,6 N Lösung (41,4 mmol) von n-Butyllithium in n-Hexan unter Rühren zugetropft. Nach Erwärmen auf 0°C für 5 Minuten wird erneut auf -78°C gekühlt und eine lösung von 6,4 g (37,7 mmol) 3-Cyclohexyl-propionsäuremethylester in 25 ml Tetrahydrofuran so zugetropft, daß die Temperatur nicht über -60°C steigt. Man laßt noch 30 Minuten bei -78°C rühren und tropft anschließend eine Lösung von 11,5 g (25,1 mmol) 2-[4-(2-n-Butyl-4-chlor-5-formyl-imidazol-1-yl-methyl)phenyl]-2-cyclopentyl-essigsäure-tert.butylester in 25 ml Tetrahydrofuran wiederum so zu, daß die Temperatur -60°C nicht übersteigt. Nach 45 Minuten bei -78°C läßt man auftauen und versetzt mit 20 ml wäßriger Ammoniumchloridlösung. Nach dreimaliger Extraktion mit Essigsäureethylester werden die vereinigten organischen Phasen über Natriumsulfat getrocknet, filtriert, eingeengt und über Kieselgel 60 mit Petrolether 40-60 / Essigsäureethylester = 6/1 chromatographiert.
Ausbeute: 11,2 g (74% der Theorie)
$R_f$ = 0,72/0,83 (Petrolether 40-60 / Essigsäureethylester = 1/1)

Beispiel II

2-[4-{2-n-Butyl-4-chlor-5-(1-(1-acetoxy-3-cyclohexyl-2-methoxycarbonyl)propyl)-1H-imidazol-1-yl-methyl}phenyl]-2-cyclopentyl-essigsäure-tert.butylester

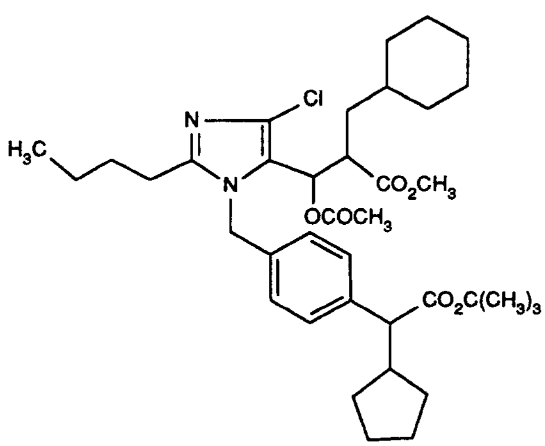

Eine Lösung von 11,0 g (17,5 mmol) der Verbindung aus Beispiel I in 300 ml Dichlormethan wird mit 0,9 g (7,2 mmol) N,N-Dimethylaminopyridin und 1,8 g (18,3 mmol) Acetanhydrid versetzt und 3 Stunden bei Raumtemperatur gerührt. Nach Zusatz von Diethylether wird die organische Phase mit Wasser, wäßriger Bicarbonat-, wäßriger Kochsalzlösung und erneut mit Wasser geschüttelt, abgetrennt, über Natriumsulfat getrocknet, filtriert und eingeengt.
Ausbeute: 11,3 g (97% der Theorie)
$R_f$ = 0,84 (Petrolether 40-60 / Essigsäureethylester = 5/4)

Herstellungsbeispiele

Beispiel 1 und Beispiel 2

(E)-2-[4-{2-n-Butyl-4-chlor-5-(1-(3-cyclohexyl-2-methoxycarbonyl)prop-1-en-yl)-1H-imidazol-1-yl-methyl}phenyl]-2-cyclopentyl-essigsäure-tert.butylester(Beispiel 1)

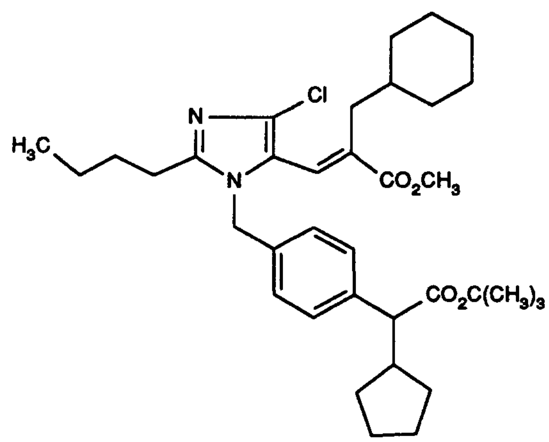

(Z)-2-[4-{2-n-Butyl-4-chlor-5-(1-(3-cyclohexyl-2-methoxycarbonyl)prop-1-en-yl)-1H-imidazol-1-yl-methyl}phenyl]-2-cyclopentyl-essigsäure-tert.butylester (Beispiel 2)

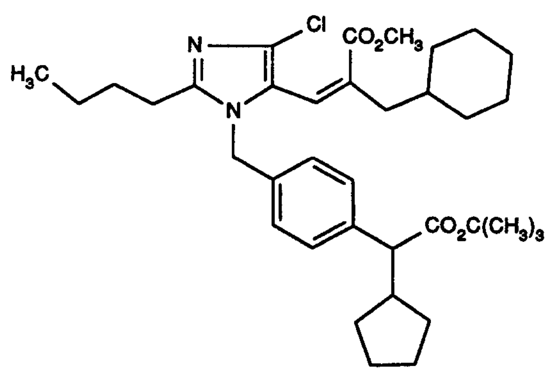

11,2 g (16,7 mmol) der Verbindung aus Beispiel II werden in 130 ml Toluol gelöst, bei Raumtemperatur mit 6,4 g (41,9 mmol) 1,8-Diazabicyclo[5,4,0]undec-7-en versetzt und 18 Stunden bei 80 °C gerührt. Nach erneutem Zusatz der gleichen Menge Base und weiteren 10 h Rühren bei 80 °C wird abgekühlt, mit Toluol verdünnt und mit wäßriger Kochsalzlösung geschüttelt. Die organische Phase wird abgetrennt, über Natriumsulfat getrocknet, filtriert, eingeengt und über Kieselgel 60 mit Petrolether 40-60 / Essigsäureethylester (4:1) chromatographiert.
Beispiel 1: Ausbeute: 4,5 g (44% der Theorie)
Beispiel 1:$R_f$ = 0,35 (Petrolether 40-60 / Essigsäureethylester = 4/1)
Beispiel 2: Ausbeute: 1,2 g (11% der Theorie)

Beispiel 2: $R_f$ = 0,21 (Petrolether 40-60 / Essigsäureethylester = 4/1)

Beispiel 3

(E)-2-[4-{2-n-Butyl-4-chlor-5-(1-(3-cyclohexyl-2-methoxycarbonyl)prop-1-en-yl)-1H-imidazol-1-yl-methyl}phenyl]-2-cyclopentyl-essigsäure

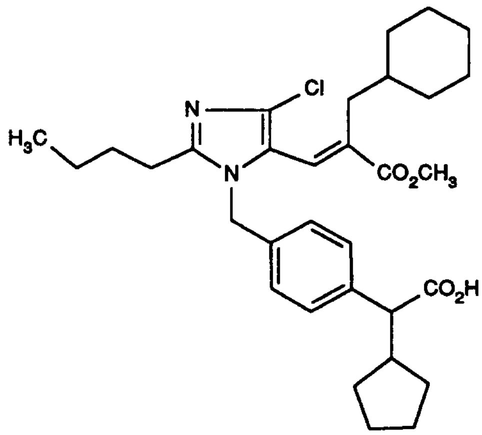

Eine Lösung von 4,4 g (7,2 mmol) der Verbindung aus Beispiel 1 in 20 ml Dichlormethan wird bei Raumtemperatur mit 20 ml Trifluoressigsäure 4 Stunden gerührt. Anschließend wird mit wäßriger Bicarbonat-Lösung alkalisch gestellt, mit Diethylether versetzt, mit wäßriger 1 N HCl auf pH 2 eingestellt, ausgeschüttelt und getrennt. Nach erneuter zweimaliger Extraktion der wäßrigen Phase mit Essigsäureethylester werden die vereinigten organischen Phasen über Natriumsulfat getrocknet, filtriert und eingeengt.
Ausbeute: 3,9 g (98% der Theorie)
$R_f$ = 0,49 (Dichlormethan / Methanol / Eisessig = 9/1/0,1)

Beispiel 4

(E)-2-[4-{2-n-Butyl-4-chlor-5-(1-(2-carboxy-3-cyclohexyl-prop-1-en-yl)-1H-imidazol-1-yl-methyl}phenyl]-2-cyclopentyl-essigsäure

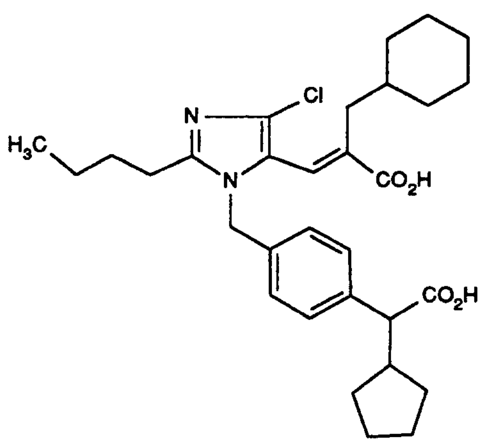

Eine Lösung von 300 mg (0,5 mmol) der Verbindung aus Beispiel 3 in 20 ml Dioxan / Wasser (1:1) wird bei Raumtemperatur mit einer Lösung von 50 mg (1,2 mmol) Lithiumhydroxid in 6 ml Wasser versetzt und 18 Stunden bei Raumtemperatur gerührt. Anschließend wird eingeengt, mit Wasser verdünnt und mit Diethylether extrahiert. Die wäßrige Phase wird mit 1 N HCl angesäuert, dreimal mit Essigsäureethylester extrahiert, und die vereinigten Essigesterphasen werden über Natriumsulfat getrocknet, filtriert und eingeengt.
Ausbeute: 274 mg (99% der Theorie)

$R_f$ = 0,28 (Dichlormethan / Methanol / Eisessig = 9/1/0,1)

Beispiel 5

(E)-2-[4-{2-n-Butyl-4-chlor-5-(3-cyclohexyl-2-methoxycarbonyl-prop-1-en-yl)-1H-imidazol-1-yl-methyl}phenyl]-2-cyclopentyl-essigsäure-L-phenylglycinolamid

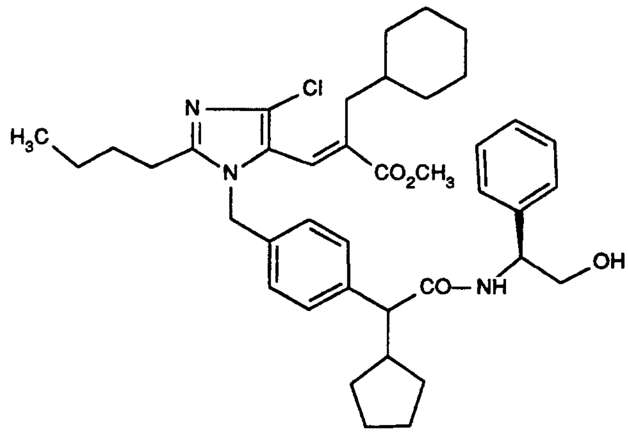

Eine Lösung von 1,2 g (2,2 mmol) der Verbindung aus Beispiel 3 in 40 ml Dichlormethan wird unter Argon bei Raumtemperatur mit 0,51 g (3,3 mmol) 1-Hydroxy-1H-benzotriazol vesetzt, auf 0 °C gekühlt und nach Zugabe von 0,44 g (4,4 mmol) Triethylamin und 0,68 g (3,3 mmol) N,N'-Dicyclohexylcarbodiimid 30 Minuten gerührt. Anschließend läßt man eine Lösung von 0,36 g (2,6 mmol) L-Phenylglycinol in 20 ml Dichlormethan zutropfen. Nach einstündigem Rühren wird langsam auf Raumtemperatur erwärmt und über Nacht gerührt. Zur Aufarbeitung wird mit Wasser versetzt und dreimal mit Dichlormethan extrahiert. Die vereinigten organischen Phasen werden über Natriumsulfat getrocknet, filtriert und über Kieselgel 60 mit Toluol / Essigsäureethylester (1:1) chromatographiert.
Ausbeute: 1,13 g (76% der Theorie)
$R_f$ = 0,50/0,55 (Dichlormethan / Methanol 9/1)

Beispiel 6

(E)-N-4-Toluolsulfonyl-2-[4-{2-n-butyl-4-chlor-5-(3-cyclohexyl-2-methoxycarbonyl-prop-1-en-yl)-1H-imidazol-1-yl-methyl}phenyl]-2-cyclopentyl-essigsäureamid

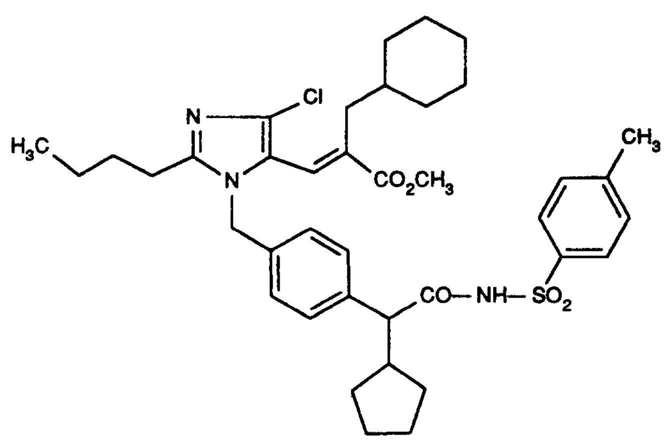

Eine Lösung von 1,2 g (2,2 mmol) der Verbindung aus Beispiel 3 in 40 ml Tetrahydrofuran wird unter Argon bei -30 °C mit 0,44 g (4,4 mmol) Triethylamin und 0,28 g (2,4 mmol) Methansulfonsäurechlorid versetzt und

2 Stunden gerührt. Anschließend läßt man eine Lösung von 0,45 g (2,6 mmol) p-Toluolsulfonamid und 0,29 g (2,2 mmol) N,N-Dimethylaminopyridin in 20 ml Tetrahydofuran zutropfen, rührt noch 1 Stunde, läßt langsam auf Raumtemperatur kommen und rührt über Nacht. Zur Aufarbeitung wird mit 10 ml 1 N Essigsäure versetzt und dreimal mit Essigsäureethylester extrahiert. Die vereinigten organischen Phasen werden über Natriumsulfat getrocknet, filtriert, eingeengt und über Kieselgel 60 mit Toluol / Essigsäureethylester (1:3) chromatographiert.

Ausbeute: 0,82 g (55% der Theorie)

$R_f$ = 0,83 (Essigsäureethylester / Petrolether 40:60 = 7/3)

Die in den Tabellen 1 und 2 aufgeführten Beispiele werden in Analogie zu den dort angegebenen Beispielen hergestellt:

Tabelle 1:

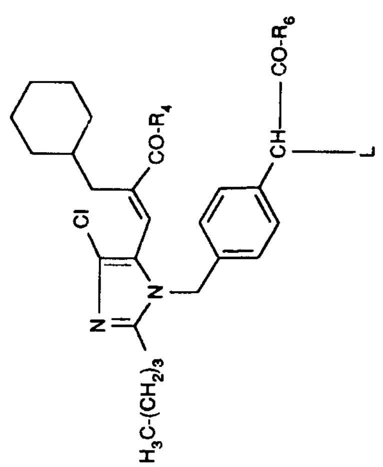

(E)

| Bsp.-Nr. | L | $R^4$ | $R^6$ | Isomer | in Analogie zur Vorschrift des Beispiels | $R_f$ (Laufmittel) |
|---|---|---|---|---|---|---|
| 7 | 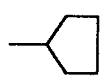 | -OH | 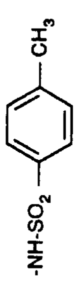 | rac | 6/4 | 0,40 C |
| 8 | 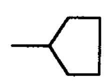 | -OH | 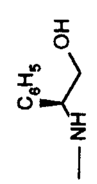 | dia | 5/4 | 0,39 C |
| 9 | $-CH_2-C_6H_5$ | -OH | OH | rac | 4 | 0,27 B |
| 10 | $-CH_2C_6H_5$ | -OH | 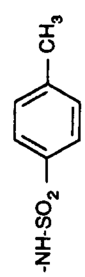 | rac | 6/4 | 0,45 B |
| 11 | $-CH_2C_6H_5$ | -OH | 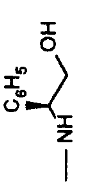 | dia | 5/4 | 0,40 B |
| 12 | 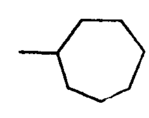 | -OH | OH | rac | 4 | 0,33 B |

Fortsetzung Tabelle 1:

| Bsp.-Nr. | L | $R^4$ | $R^6$ | Isomer | in Analogie zur Vorschrift des Beispiels | $R_f$ (Laufmittel) |
|---|---|---|---|---|---|---|
| 13 | $-CH(CH_3)_2$ | -OH | -OH | rac | 4 | 0,44 C |
| 14 | $-CH(CH_3)_2$ | $-OCH_3$ | 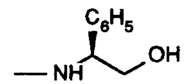 | dia A | 5 | 0,46 D |
| 15 | $-CH(CH_3)_2$ | $-OCH_3$ | 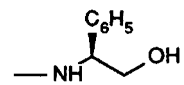 | dia B | 5 | 0,35 D |
| 16 | $-CH(CH_3)_2$ | -OH | 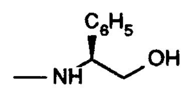 | dia A | 5/4 | 0,26 B |
| 17 | $-CH(CH_3)_2$ | -OH | 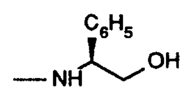 | dia B | 5/4 | 0,37 B |
| 18 | $-CH(CH_3)_2$ | -OH | 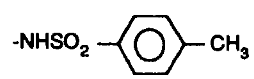 | rac | 6/4 | 0,43 B |

Tabelle 2:

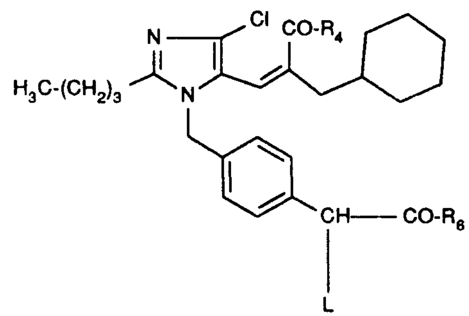

(Z)

| Bsp.-Nr. | L | $R^4$ | $R^6$ | Isomer | in Analogie zur Vorschrift des Beispiels | $R_f$ (Laufmittel) |
|---|---|---|---|---|---|---|
| 19 | $-CH(CH_3)_2$ | -OH | OH | rac | 4 | 0,47 C |
| 20 | $-CH_2C_6H_5$ | -OH | OH | dia | 4 | 0,45 C |
| 21 | 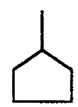 | -OH | OH | rac | 4 | 0,44 C |
| 22 | 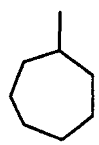 | -OH | OH | rac | 4 | 0,44 C |
| 23 | 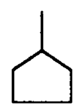 | $-OCH_3$ | 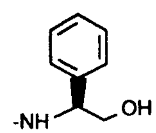 | dia | 5 | 0,74/0,70 A |

Fortsetzung Tabelle 2:

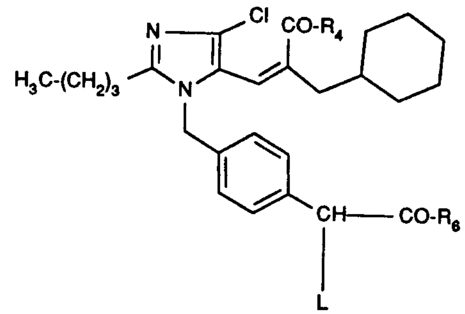

(Z)

| Bsp.-Nr. | L | $R^4$ | $R^6$ | Isomer | in Analogie zur Vorschrift des Beispiels | $R_f$ (Laufmittel) |
|---|---|---|---|---|---|---|
| 24 | 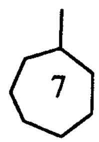 | $-OCH_3$ | 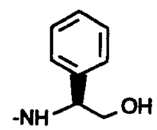 | dia | 5 | 0,72 A |
| 25 | 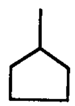 | -OH | 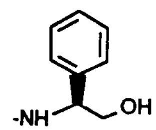 | dia | 5/4 | 0,08 A |
| 26 | 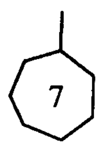 | -OH | 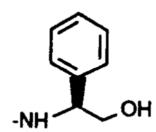 | dia | 5/4 | 0,12 A |

Fortsetzung Tabelle 2:

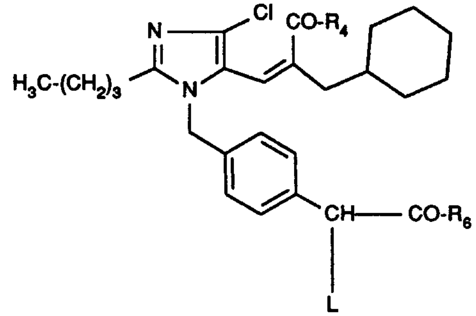

(Z)

| Bsp.-Nr. | L | $R^4$ | $R^6$ | Isomer | in Analogie zur Vorschrift des Beispiels | $R_f$ (Laufmittel) |
|---|---|---|---|---|---|---|
| 27 | 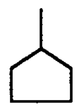 | -OH | 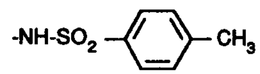 | rac | 6/4 | 0,47 B |
| 28 | 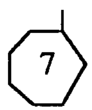 | -OH | 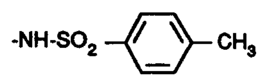 | rac | 6/4 | 0,46 B |

## Patentansprüche

1. Propenoyl-imidazolderivate der Formel (I)

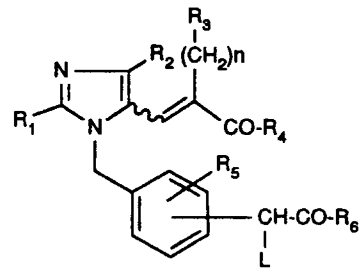

(I)

in welcher

| | |
|---|---|
| $R^1$ | für geradkettiges oder verzweigtes Alkyl oder Alkenyl mit jeweils bis zu 8 Kohlenstoffatomen steht, oder für Cycloalkyl mit 3 bis 8 Kohlenstoffatomen steht, |
| $R^2$ | für Wasserstoff, Halogen oder für Perfluoralkyl mit bis zu 5 Kohlenstoffatomen steht, |
| n | für eine Zahl 0, 1, 2 oder 3 steht, |
| $R^3$ | für Cycloalkyl mit 3 bis 7 Kohlenstoffatomen steht, |
| $R^4$ | für Hydroxy, für geradkettiges oder verzweigtes Alkoxy mit bis zu 6 Kohlenstoffatomen steht, oder für einen Rest der Formel $-CO-NR^7R^8$ steht, worin |
| $R^7$ und $R^8$ | gleich oder verschieden sind und Wasserstoff, geradkettiges oder verzweigtes Alkyl mit bis zu 6 Kohlenstoffatomen oder Phenyl bedeuten, |
| $R^5$ | für Wasserstoff, Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano oder Carboxy steht, |
| L | für Cycloalkyl mit 3 bis 7 Kohlenstoffatomen steht, oder für geradkettiges oder verzweigtes Alkyl mit bis zu 8 Kohlenstoffatomen steht, das gegebenenfalls durch Phenyl substituiert ist, |
| $R^6$ | für Hydroxy oder geradkettiges oder verzweigtes Alkoxy mit bis zu 6 Kohlenstoffatomen steht, oder für einen Rest der Formel $-NR^9SO_2R^{10}$ oder 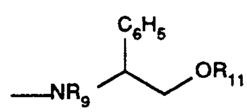 steht, worin |
| $R^9$ | Wasserstoff oder geradkettiges oder verzweigtes Alkyl mit bis zu 4 Kohlenstoffatomen bedeutet, |
| $R^{10}$ | geradkettiges oder verzweigtes Alkyl mit bis zu 6 Kohlenstoffatomen oder Phenyl bedeutet, das gegebenenfalls durch geradkettiges oder verzweigtes Alkyl mit bis zu 6 Kohlenstoffatomen substituiert ist, |
| $R^{11}$ | Wasserstoff, geradkettiges oder verzweigtes Alkyl mit bis zu 6 Kohlenstoffatomen oder eine Hydroxyschutzgruppe bedeutet |

und deren Salze.

**2.** Propenoyl-imidazolderivate nach Anspruch 1, wobei

| | |
|---|---|
| $R^1$ | für geradkettiges oder verzweigtes Alkyl oder Alkenyl mit jeweils bis zu 6 Kohlenstoffatomen steht, oder für Cyclopropyl, Cyclobutyl, Cyclopentyl, Cyclohexyl oder Cycloheptyl steht, |
| $R^2$ | für Wasserstoff, Fluor, Chlor, Brom oder für Perfluoraikyl mit bis zu 4 Kohlenstoffato- |

men steht,

n für eine Zahl 0, 1 oder 2 steht,

$R^3$ für Cyclopropyl, Cyclobutyl, Cyclopentyl, Cyclohexyl oder Cycloheptyl steht,

$R^4$ für Hydroxy, für geradkettiges oder verzweigtes Alkoxy mit bis zu 4 Kohlenstoffatomen, oder
für die Gruppe der Formel $-CO-NR^7R^8$ steht,
worin

$R^7$ und $R^8$ gleich oder verschieden sind und Wasserstoff, geradkettiges oder verzweigtes Alkyl mit bis zu 4 Kohlenstoffatomen oder Phenyl bedeuten,

$R^5$ für Wasserstoff, Fluor, Chlor, Brom, Trifluormethyl, Carboxy oder geradkettiges oder verzweigtes Alkyl, Alkoxy oder Alkoxycarbonyl mit jeweils bis zu 4 Kohlenstoffatomen steht,

L für Cyclopropyl, Cyclobutyl, Cyclopentyl, Cyclohexyl oder Cycloheptyl steht, oder
für geradkettiges oder verzweigtes Alkyl mit bis zu 6 Kohlenstoffatomen oder für Benzyl steht,

$R^6$ für Hydroxy oder für geradkettiges oder verzweigtes Alkoxy mit bis zu 4 Kohlenstoffatomen steht, oder
für einen Rest der Formel $-NR^9SO_2R^{10}$ oder

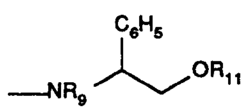

steht,
worin

$R^9$ Wasserstoff, Methyl oder Ethyl bedeutet,

$R^{10}$ geradkettiges oder verzweigtes Alkyl mit bis zu 4 Kohlenstoffatomen oder Phenyl bedeutet, das gegebenenfalls durch geradkettiges oder verzweigtes Alkyl mit bis zu 4 Kohlenstoffatomen substituiert ist,

$R^{11}$ Wasserstoff, geradkettiges oder verzweigtes Alkyl mit bis zu 4 Kohlenstoffatomen, Acetyl oder Benzyl bedeutet,

und deren Salze.

**3.** Propenoyl-imidazolderivate nach Anspruch 1,
wobei

$R^1$ für geradkettiges oder verzweigtes Alkyl oder Alkenyl mit jeweils bis zu 4 Kohlenstoffatomen steht, oder
für Cyclopropyl, Cyclopentyl oder Cyclohexyl steht,

$R^2$ für Wasserstoff, Fluor, Chlor oder für Perfluoralkyl mit bis zu 2 Kohlenstoffatomen steht,

n für die Zahl 0 oder 1 steht,

$R^3$ für Cyclopentyl oder Cyclohexyl steht,

$R^4$ für Hydroxy, Methoxy, Ethoxy oder tert.Butoxy steht,

$R^5$ für Wasserstoff, Fluor, Chlor, Brom, Trifluormethyl, Trifluormethoxy oder Methyl steht,

L für Cyclopentyl, Cyclohexyl oder Cycloheptyl steht, oder für geradkettiges oder verzweigtes Alkyl mit bis zu 4 Kohlenstoffatomen oder für Benzyl steht,

$R^6$ für Hydroxy oder für geradkettiges oder verzweigtes Alkoxy mit bis zu 4 Kohlenstoffatomen steht, oder
für einen Rest der Formel $-NR^9SO_2R^{10}$ oder

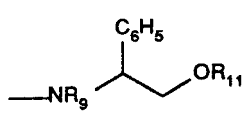

steht,
worin

$R^9$ Wasserstoff oder Methyl bedeutet,
$R^{10}$ Methyl oder p-Tolyl bedeutet,
$R^{11}$ Wasserstoff oder geradkettiges oder verzweigtes Alkyl mit bis zu 3 Kohlenstoffatomen oder Benzyl bedeutet,

und deren Salze.

4. Propenoyl-imidazolderivate nach Anspruch 1 in welchen der Rest -CH(L)-CO-$R^4$ in para-Position zur Imidazolylmethylgruppe steht.

5. Propenoyl-imidazolderivate nach Anspruch 1 zur therapeutischen Anwendung.

6. Verfahren zur Herstellung von Propenoyl-imidazolderivaten nach Anspruch 1, dadurch gekennzeichnet, daß man Aldehyde der allgemeinen Formel (II)

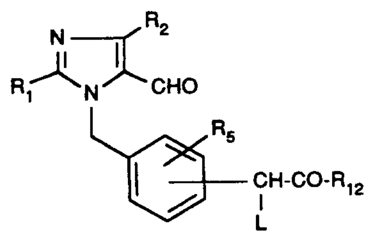

(II)

in welcher
$R^1$, $R^2$, $R^5$ und L die in Anspruch 1 angegebene Bedeutung haben
und
$R^{12}$ für $C_1$-$C_4$-Alkoxy steht,
durch Umsetzung mit Verbindungen der allgemeinen Formel (III)

$R^3$-$(CH_2)_n$-$CH_2$-CO-$R^{13}$ (III)

in welcher
$R^3$ und n die in Anspruch 1 angegebene Bedeutung haben
und
$R^{13}$ die in Anspruch 1 angegebene Bedeutung von $R^4$ hat aber nicht für Hydroxy steht,
in inerten Lösemitteln, in Anwesenheit einer Base in die Hydroxy-Verbindungen der allgemeinen Formel (IV)

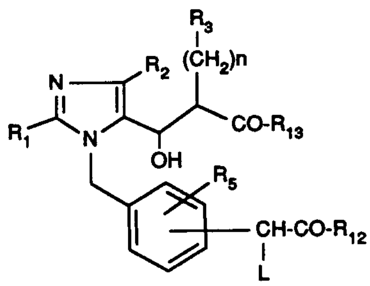

(IV)

in welcher
$R^1$, $R^2$, $R^3$, $R^5$, $R^{12}$, $R^{13}$, n und L die in Anspruch 1 angegebene Bedeutung haben,
überführt,
anschließend die freie Hydroxyfunktion durch Einführung einer Schutzgruppe blockiert, in einem letzten Schritt eine Eliminierung in inerten Lösemitteln, in Anwesenheit einer Base durchführt, gegebenenfalls die E/Z-Isomeren trennt,
und im Fall der Säuren ($R^4$ = OH) die Ester verseift,

und im Fall der Amide oder Sulfonamide, gegebenenfalls nach Aktivierung der Carbonsäure ($R^{12}$ = OH) eine Amidierung oder Sulfoamidierung nach üblichen Methoden anschließt,
im Fall, daß $R^9$ nicht für Wasserstoff steht die NH-Funktion alkyliert,
und gegebenenfalls die Substituenten $R^1$ und $R^2$ nach üblichen Methoden, beispielsweise durch Reduktion, Oxidation, Alkylierung oder Hydrolyse einführt oder in andere Gruppen überführt
und gegebenenfalls die Stereoisomeren trennt, und im Fall der Herstellung der Salze mit einer entsprechenden Base oder Säure umsetzt.

**7.** Arzneimittel enthaltend mindestens ein Propenoyl-imidazolderivat nach Anspruch 1.

**8.** Arzneimittel nach Anspruch 7 zur Behandlung von arterieller Hypertonie und Atherosklerose.

**9.** Verwendung der Propenoyl-imidazolderivate nach Anspruch 1 zur Herstellung von Arzneimitteln.

**10.** Verwendung nach Anspruch 9 zur Herstellung von Arzneimitteln zur Behandlung von arterieller Hypertonie und Atherosklerose.